# EUROPEAN PATENT SPECIFICATION

(11) **EP 4 452 152 B1**
(45) Date of publication and mention of the grant of the patent: **26.08.2026**
(21) Application number: 22840017.2
(22) Date of filing: 21.12.2022
(51) Int. Cl.: A61F 5/445

(54) **OSTOMY APPLIANCE**
OSTOMIEVORRICHTUNG
APPAREIL DE STOMIE

(30) Priority: 21.12.2021 DK PA202170641
(43) Date of publication of application: 30.10.2024
(73) Proprietor: Coloplast A/S, 3050 Humlebaek (DK)
(72) Inventor: MOLZEN, Lars, 3450 Alleroed (DK); SUND, Anders Grove, 3450 Alleroed (DK); HANSEN, Kristoffer, 2850 Naerum (DK)
(86) International application number: PCT/DK2022/050302
(87) International publication number: WO 2023/117020

(56) References cited:
- WO-A1-2007/098762
- WO-A1-2020/252458
- US-A1- 2020 246 175

## Description

The present disclosure relates to a medical device, and in particular to an ostomy appliance for attachment to the skin surface of a user. In particular, the present disclosure relates to a sensing device for sensing of moisture in the appliance, the sensing being based on wicking from regions of the appliance to an interface with electrodes. Also disclosed is an ostomy system for sensing of moisture in an ostomy appliance.

### Brief description of the drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 illustrates an exemplary exploded perspective view of an ostomy appliance according to an embodiment of the invention;
Fig. 2A illustrates an exemplary cross-sectional view of an ostomy appliance according to an embodiment of the invention;
Fig. 2B illustrates an exemplary cross-sectional view of an ostomy appliance according to an embodiment of the invention;
Fig. 3 illustrates a perspective view of distal surface of the nonwoven substrate with a plurality of wicking zones according to an embodiment of the invention;
Fig. 4 illustrates an exemplary situation of two instances of liquid wetting a nonwoven substrate according to an embodiment of the invention; and
Fig. 5 illustrates a nonwoven layer according to an embodiment of the invention.

### Background

WO 2007/098762 A1 relates to a method and dressing for detecting detachment of the dressing, which is applied to a surface of an at least partly electrically conductive object. The dressing comprises an adhesive and at least two electrodes arranged in a distance from the electrically conductive object. Changes in capacitance are detected, and an alarm is activated when the changes of the capacitance reach a predetermined value.

### Detailed description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output," "waste(s)," "liquids," and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated, or be implicit from the context that the "user" is not the "patient" him- or herself.

In the following, whenever referring to proximal side or surface of a layer, an element, a device or part of a device, the referral is to the skin-facing side or surface, when a user wears the ostomy appliance. Likewise, whenever referring to the distal side or surface of a layer, an element, a device or part of a device, the referral is to the side or surface facing away from the skin, when a user wears the ostomy appliance. In other words, the proximal side or surface is the side or surface closest to the user, when the appliance is fitted on a user and the distal side is the opposite side or surface - the side or surface furthest away from the user in use.

The axial direction is defined as the direction of the stoma, when a user wears the appliance. Thus, the axial direction is generally perpendicular to the skin or abdominal surface of the user.

A radial direction is defined as perpendicular to the axial direction. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The present disclosure provides an ostomy appliance for attachment to the skin surface of a user and an ostomy system comprising the ostomy appliance and a monitor device.

The present disclosure relates to an ostomy system and devices thereof, such as an ostomy appliance, a base plate for an ostomy appliance, a sensor patch for application to a base plate, a monitor device, and optionally one or more accessory devices. Further, methods related to the ostomy system and devices thereof are disclosed. An accessory device (also referred to as an external device) can be a mobile phone or other handheld device, such as a smart device including a smartphone or a smartwatch. In embodiments, an accessory device is a personal electronic device, e.g., a wearable, such as a watch or other wrist-worn electronic device. An accessory device can be a docking station. In embodiments, the docking station is configured to electrically and/or mechanically couple the monitor device to the docking station. In embodiments, the docking station is configured for charging a battery of the monitor device and/or configured for transferring data between the monitor device and the docking station. The ostomy system can comprise a server device. In embodiments, the server device is operated and/or controlled by the ostomy appliance manufacturer and/or a service centre.

The present disclosure provides an ostomy system and devices thereof, such as an ostomy appliance, a base plate for an ostomy appliance, a sensor patch for application to a base plate, a monitor device, and optionally one or more accessory devices which either alone or together facilitate reliable determination of the nature, severity, and rapidness of moisture propagation in the adhesive material provided for attaching the base plate and/or sensor patch to the skin surface of a user. Depending on the nature of the pattern of moisture propagation in the adhesive, the ostomy system and devices thereof enable providing information to the user about the type of failure, such as adhesive failure patterns, and in turn enable providing an indication to the user of the severity and thus the remaining time frame for replacing the ostomy appliance without experiencing severe leakage and/or skin damage.

In embodiments, the ostomy appliance includes a base plate, such as a monolithic, one-piece base plate, e.g., integrated with a sensor assembly part, or a separate sensor assembly part, such as a sensor assembly part to be subsequently applied to a base plate. In embodiments, the sensor assembly part is a sensor patch for application to the base plate, such as the proximal surface of the base plate. Thereby, an arbitrary base plate, such as a conventional base plate provided with the sensor patch, can achieve the features as described herein. Features as described with respect to sensing/monitoring capabilities of the base plate herein can be provided by a sensor assembly of a sensor patch to be applied to a base plate, e.g., by the user, and vice versa. In embodiments, the sensor patch is adapted to adhere to a base plate. In embodiments, the sensor patch comprises a adhesive layer, such as a first adhesive layer, adapted to adhere to the skin surface of a user.

In embodiments, a method of attaching a base plate having sensing capabilities, e.g., through the provision of a sensor patch, to a user's stoma and/or skin surrounding the stoma, such as the peristomal skin area, comprises attaching the sensor patch to a base plate and attaching the base plate, i.e., together with the attached sensor patch, to the user's stoma and/or skin surrounding the stoma, such as the peristomal skin area. Alternatively, the method of attaching the base plate to the user's stoma and/or skin surrounding the stoma comprises attaching the sensor patch to the user's stoma and/or skin surrounding the stoma and attaching the base plate to the user's stoma and/or skin surrounding the stoma above the attached sensor patch, i.e., on a distal surface of the sensor patch.

In embodiments, the ostomy appliance comprises a base plate and an ostomy pouch (also referred to as an ostomy bag). The ostomy appliance can be a colostomy appliance, an ileostomy appliance, or a urostomy appliance. In embodiments, the ostomy appliance is a two-part ostomy appliance, i.e., the base plate and the ostomy pouch are releasably coupled, e.g., with a mechanical and/or an adhesive coupling, e.g., to allow that a plurality of ostomy pouches can be utilized (exchanged) with one base plate. Further, a two-part ostomy appliance can facilitate correct application of the base plate to skin, e.g., to an improved user sight of the stomal region. In embodiments, the ostomy appliance is a one-part ostomy appliance, i.e., the base plate and the ostomy pouch are fixedly attached to each other. The base plate is configured for coupling to a user's stoma and/or skin surrounding the stoma, such as a peristomal skin area.

In embodiments, the ostomy system comprises a base plate or a sensor patch for application to a base plate. In embodiments, parts of the ostomy system are incorporated into a base plate or a sensor patch for application to a base plate. In embodiments, the first adhesive layer of the ostomy system is a first adhesive layer of a base plate or a sensor patch for attachment to a base plate. In embodiments, the first adhesive layer and the sensor assembly of the ostomy system are incorporated into a base plate or sensor patch to provide such with the ability to indicate a site of leakage. In embodiments, the ostomy system provides a base plate and/or a sensor patch with the ability to indicate a site of leakage from an ostomy appliance, e.g., from the base plate.

Providing a base plate having sensing capabilities, e.g., through an incorporated sensor assembly or through a sensor patch comprising a sensor assembly provides for an optimum or improved use of an ostomy appliance. In particular, it is facilitated that a base plate is not changed too late (leading to adhesive failure, leakage, and/or skin damage), or at least that a user is informed that a leakage will happen, is happening, or has happened. Accordingly, the user or a health care professional is able to monitor and plan the use of the ostomy appliance.

In a first aspect of the invention, an ostomy appliance is disclosed.

In embodiments, the ostomy appliance comprises:
- an adhesive layer, such as a first adhesive layer, having a proximal side including a proximal surface for adhering the ostomy appliance to the skin surface, and a distal side including a distal surface;
- a nonwoven substrate arranged on the distal side of the adhesive layer; and
- a monitor interface for electrical coupling with a monitor device, the monitor interface comprising a plurality of sensing electrodes and a plurality of terminals, the plurality of sensing electrodes including a first electrode and a second electrode configured to detect presence of liquid in the non-woven substrate and the plurality of terminals including a first terminal coupled to the first electrode and configured to electrically couple with a corresponding terminal of the monitor device and a second terminal coupled to the second electrode and configured to electrically couple with a corresponding terminal of the monitor device.

The nonwoven substrate is configured to wick or guide liquid from a first region of the nonwoven substrate to at least the first electrode.

In embodiments, the ostomy appliance comprises:
- an adhesive layer having a proximal side including a proximal surface for adhering the sensor patch to the skin, and a distal side including a distal surface;
- a plurality of electrical conductors positioned in a liquid detection zone; and
- a layer of nonwoven substrate arranged on the distal side of the adhesive layer, the layer of nonwoven substrate being liquid permeable and adapted to guide liquid, such as liquid absorbed by the adhesive layer, towards said liquid detection zone.

In embodiments, the liquid detection zone may be denoted sensing region.

In embodiments, the liquid detection zone comprises a monitor interface. In the following, when the ability of the monitor interface to detect liquid is discussed, a reference to the monitor interface may be a reference to the liquid detection zone. In other words, the references to a monitor interface and a liquid detection zone may be used interchangeably in the context of the detection of liquid in the monitor interface or liquid detection zone.

In the following, the words wick, guide, transport, and similar words, are all intended to describe the ability of the nonwoven substrate to guide liquid or moisture from one region to another, preferably by means of capillary effects or osmosis.

The first aspect of the invention provides a device with a particular robust design, as the sensing circuit involving electrodes may be provided in the monitor interface area (liquid detection zone) only, whereby the majority of the ostomy appliance is absent of electrodes, which may otherwise be prone to rupture or failure.

In embodiments, the plurality of electrical conductors comprises a first sensing electrode and a second sensing electrode configured to detect presence of liquid in the nonwoven substrate.

In embodiments, the ostomy appliance further comprises a plurality of terminals positioned in the liquid detection zone/monitor interface. The plurality of terminals comprises a first terminal coupled to the first electrode and configured to electrically couple with a corresponding terminal of a monitor device and a second terminal coupled to the second electrode and configured to electrically couple with a corresponding terminal of the monitor device.

In embodiments, the nonwoven substrate is adapted to guide liquid towards the liquid detection zone by the fibres of the nonwoven substrate being aligned towards the liquid detection zone.

The adhesive layer may also be denoted the first adhesive layer, whereas explicit reference is made when referring to a second adhesive layer.

In embodiments, the adhesive layer, such as a first adhesive layer, is made of a first composition. In embodiments, the first composition comprises one or more polyisobutenes and/or styrene-isoprene-styrene. In embodiments, the first composition comprises one or more hydrocolloids. In embodiments, the first composition comprises one or more water soluble or water swellable hydrocolloids. In embodiments, the first composition is a pressure sensitive adhesive composition suitable for medical purposes comprising a rubbery elastomeric base and one or more water soluble or water swellable hydrocolloids. In embodiments, the first composition comprises one or more polybutenes, one or more styrene copolymers, one or more hydrocolloids, or any combination thereof. The combination of the adhesive properties of the polybutenes and the absorbing properties of the hydrocolloids renders the first composition suitable for use in ostomy appliances. For example, the styrene copolymer can be a styrene-butadiene-styrene block copolymer or a styrene-isoprene-styrene block copolymer. Preferably, one or more styrene-isoprene-styrene (SIS) block type copolymers are employed. The amount of styrene block-copolymer can be from 5% to 20% of the total adhesive composition. The butene component is suitably a conjugated butadiene polymer selected from polybutadiene, polyisoprene. The polybutenes are preferably present in an amount of from 35 - 50% of the total adhesive composition. Preferably, the polybutene is polyisobutylene (PIB). Suitable hydrocolloids for incorporation in the first composition are selected from naturally occurring hydrocolloids, semisynthetic hydrocolloids, and synthetic hydrocolloids. The first composition can comprise 20-60% hydrocolloids. A preferred hydrocolloid is carboxymethyl cellulose (CMC). Optionally, the first composition can contain other components, such as fillers, tackifiers, plasticizers, and/or other additives.

The adhesive layer can have a substantially uniform thickness. The adhesive layer can have a thickness in the range from 0.08 mm to 1.5 mm, e.g., in the range from 0.1 mm to 0.5 mm, thereby providing a thin adhesive, such that moisture may quickly reach the nonwoven substrate. Alternatively, the adhesive layer may have a thickness in the range between 0.8 mm and 1.0 mm. The adhesive layer can have a primary thickness in a primary part of the adhesive layer, e.g., in a primary region within a primary radial distance or in a primary radial distance range from the centre point of the stomal opening. The primary radial distance can be in the range from 20 mm to 50 mm, such as in the range from 25 mm to 35 mm, e.g., 30 mm. The adhesive layer can have a secondary thickness in a secondary part of the adhesive layer, e.g., in a secondary region outside a secondary radial distance or in a secondary radial distance range from the centre point of the stomal opening. The secondary radial distance can be in the range from 20 mm to 50 mm, such as in the range from 25 mm to 35 mm, e.g., 30 mm. The secondary thickness may be smaller than the primary thickness.

In embodiments, the ostomy appliance comprises a second layer. In embodiments, the second layer is an adhesive layer, such as a second adhesive layer. In embodiments, the second layer has a second radial extension that is larger than a first radial extension of the first adhesive layer at least in a first angular range of the base plate and/or the sensor patch. Accordingly, a part of a proximal surface of the second layer can be configured for attachment to the skin surface of a user. The part of a proximal surface of the second layer configured for attachment to the skin surface of a user is also denoted the skin attachment surface of the second adhesive layer. The second layer can have a stomal opening, such as a second layer stomal opening and/or a second adhesive stomal opening, with a centre point.

In embodiments, the second adhesive layer is made of a second composition. In embodiments, the second composition comprises one or more polyisobutenes and/or styrene-isoprene-styrene. In embodiments, the second composition comprises one or more hydrocolloids. In embodiments, the second composition comprises one or more water soluble or water swellable hydrocolloids. In embodiments, the second composition is a pressure sensitive adhesive composition suitable for medical purposes comprising a rubbery elastomeric base and one or more water soluble or water swellable hydrocolloids. In embodiments, the second composition comprises one or more polybutenes, one or more styrene copolymers, one or more hydrocolloids, or any combination thereof. The combination of the adhesive properties of the polybutenes and the absorbing properties of the hydrocolloids renders the second composition suitable for use in ostomy appliances. For example, the styrene copolymer can be a styrene-butadiene-styrene block copolymer or a styrene-isoprene-styrene block copolymer. Preferably, one or more styrene-isoprene-styrene (SIS) block type copolymers are employed. The amount of styrene block-copolymer can be from 5% to 20% of the total adhesive composition. The butene component is suitably a conjugated butadiene polymer selected from polybutadiene, polyisoprene. The polybutenes are preferably present in an amount of from 35 - 50% of the total adhesive composition. Preferably, the polybutene is polyisobutylene (PIB). Suitable hydrocolloids for incorporation in the second composition are selected from naturally occurring hydrocolloids, semisynthetic hydrocolloids, and synthetic hydrocolloids. The second composition can comprise 20-60% hydrocolloids. A preferred hydrocolloid is carboxymethyl cellulose (CMC). Optionally, the second composition can contain other components, such as fillers, tackifiers, plasticizers, and/or other additives.

Different ratio of contents can change properties of the first and/or second adhesive layers. In embodiments, the second adhesive layer and the first adhesive layer have different properties. In embodiments, the second adhesive layer (second composition) and the first adhesive layer (first composition) have different ratios of polyisobutenes, styrene-isoprene-styrene, and/or hydrocolloids. For example, the second adhesive layer can provide a stronger attachment to the skin compared to attachment to the skin provided by the first adhesive layer. Alternatively, or additionally, the second adhesive layer can be thinner than the first adhesive layer. Alternatively, or additionally, the second adhesive layer can be less water and/or sweat absorbing than the first adhesive layer. Alternatively, or additionally, the second adhesive layer can be less mouldable than the first adhesive layer. In embodiments, the second adhesive layer provides a second barrier against leakage.

The second layer can have a substantially uniform thickness similar to that of the first adhesive layer.

The ostomy appliance comprises a nonwoven substrate arranged on the distal side of the first adhesive layer. By a nonwoven substrate is meant a nonwoven textile fabric, i.e., a textile comprising a plurality of intertwined fibres made according to common nonwoven fabrication techniques, such as spunlaid/spunbond nonwovens or melt-blown nonwovens, although the use of other types of nonwovens are foreseen. In other words, by a nonwoven fabric or textile is meant a material made from fibres, such as stable fibres and/or long fibres, bonded together (entangled) by chemical, mechanical, heat or solvent treatment.

For example, the nonwoven substrate is arranged on the distal surface of the first adhesive layer. Since the first adhesive layer is moisture absorbing, such moisture will eventually reach the nonwoven substrate arranged on the distal side of said first adhesive layer. Once the moisture reaches the nonwoven substrate, it will be adsorbed (i.e., bonded on the surface, as opposed to absorbed) by said nonwoven substrate and transported, by wicking (capillary action), to areas of less moisture content (osmosis) - e.g., to the monitor interface.

The nonwoven substrate is configured to wick/guide liquid from a first region of the substrate to at least the first electrode. In other words, the properties of the nonwoven substrate are chosen to facilitate moisture/liquid to be transported to the at least first electrode arranged a liquid detection zone, such as in the monitor interface, and coupled to the first terminal. The first region may be a defined area of the ostomy appliance in the appliance plane spanned by ostomy appliance (e.g., the first adhesive layer and the nonwoven substrate).

The nonwoven substrate should at least exhibit wicking to facilitate the transport/guiding of liquid/moisture from the first region to the liquid detection zone or monitor interface. In a preferred embodiment, the nonwoven substrate comprises adsorbent fibres (e.g., as opposed to absorbent fibres), thereby providing efficient capillary action to the liquid. In a preferred embodiment, the nonwoven substrate comprises hydrophobic fibres. In embodiments, the fibres of the nonwoven substrate may be natural fibres, such as wool. In embodiments, the fibres of the nonwoven substrate may be synthetic fibres. In a preferred embodiment, the synthetic fibres are non-porous and hydrophobic to substantially hinder the absorption of liquid, but to facilitate efficient adsorption. In embodiments, the nonwoven substrate comprises synthetic fibres made of one or more of polypropylene (PP), polyester (PE), polyurethane (PU), polyethylene terephthalate (PET), polyethersulfone (PES) and polyamide/nylon (PA). For example, the nonwoven substrate may comprise one or more (a blend) of said synthetic fibres.

In embodiments, the nonwoven substrate has a weight (area density) between 10 g/m² and 40 g/m². The weight/area density may be measured according to ISO 9073-1. In embodiments, the nonwoven substrate has a thickness between 75 µm and 270 µm. The thickness may be measured according to ISO 9073-2.

The ostomy appliance comprises a monitor interface for electrical coupling with a monitor device. The monitor interface may comprise coupling means for detachably fixating the monitor device relative to the monitor interface. In embodiments, the monitor interface comprises one or more sensing electrodes and one or more terminals configured to electrically couple the one or more sensing electrodes with corresponding terminals of a monitor device. In a preferred embodiment, the monitor interface comprises a plurality of sensing electrodes including a first electrode and a second electrode configured to detect presence of liquid in the nonwoven substrate. Thus, by means of the sensing electrodes, liquid may be detected in the immediate vicinity of the monitor interface, such that the electrodes do not need to extend into regions of the ostomy appliance as such. Thereby, electrodes may only be present in/within the monitor interface, which thus constitutes the liquid detection zone as referred to above. The electrodes may be configured to detect presence of liquid in the nonwoven substrate by being exposed to said nonwoven substrate, such that the electrodes may measure an electrical quantity (e.g., resistance, conductance, impedance, or capacitance) of the nonwoven substrate in the vicinity of the monitor interface/liquid detection zone. The monitor interface further comprises a plurality of terminals including a first terminal and a second terminal. The first terminal is configured to electrically couple with a corresponding terminal of an attached monitor device. The second terminal is configured to electrically couple with a corresponding terminal of an attached monitor device. Further, the first terminal is coupled to the first electrode, such that readings (e.g., conducted by the monitor device) of the first terminal is indicative of the surroundings of the first electrode. Likewise, the second terminal is coupled to the second electrode, such that readings (e.g., conducted by the monitor device) of the second terminal is indicative of the surroundings of the second electrode. The electrodes and/or the terminals may comprise one or more of metallic (e.g. silver, copper, gold, titanium, aluminium, stainless steel), ceramic (e.g. ITO), polymeric (e.g. PEDOT, PANI, PPy), and carbonaceous (e.g. carbon black, carbon nanotube, carbon fibre, graphene, graphite) materials.

In embodiments, a sensing electrode and the corresponding terminal is combined, such that liquid may be detected directly by the terminal, which is also configured to connect to a monitor device. Thereby, the design of the device may be simplified. For example, the terminals/sensing electrodes may be formed as planar pads, where a proximal surface of a pad is in contact with the nonwoven substrate and a distal surface of the same pad forms the corresponding terminal.

Thereby, following an incident (e.g., sweating or presence of output in the interface between the skin surface and the proximal surface of the first adhesive layer) where liquid is absorbed by the first adhesive layer and transported through, to reach the nonwoven substrate, said liquid may be transported, by wicking, through the nonwoven substrate to reach the monitor interface, where the presence of liquid may be determined by means of the sensing electrodes once coupled to a monitor device via the terminals of said monitor interface. Thereby is provided an ostomy appliance for detection of liquid, which is robust and flexible in the majority of the appliance, and where the electrodes are only to be present in the monitor interface, which may be arranged distant from a stomal opening of the ostomy appliance.

In an embodiment, the nonwoven substrate is divided into a plurality of wicking zones including a first wicking zone, the first wicking zone being in communication with at least the first electrode, such that liquid within the first wicking zone may be transported to said first electrode. Thus, by being in communication is meant that liquid may be transported/wicked/guided from anywhere (any point) within the first wicking zone to at least the first electrode of the monitor interface for detection. Similarly, the plurality of wicking zones may include a second wicking zone, the second wicking zone being in communication with at least the second electrode, such that liquid within the second wicking zone may be transported to said second electrode for detection. Thus, the plurality of wicking zones may comprise two or more wicking zones, such as two, three, four, five, six, seven, eight or more wicking zones. Each wicking zone of the plurality of wicking zones is in communication with at least one electrode of the monitor interface, such that liquid occurring within a zone may be transported (by wicking) to the monitor interface for detection. The wicking zones facilitate narrowing down where liquid is present. In other words, by providing two or more wicking zones, it may be determined where liquid is occurring in the ostomy appliance based on in which zone liquid is determined in the monitor interface.

In embodiments, one or more, such as two, sensing electrodes are associated with each wicking zone, such that liquid in a certain wicking zone may be sensed by means of such one or more, such as two, sensing electrodes.

In an embodiment, transport of liquid between wicking zones of the plurality of wicking zones is inhibited. Thereby, liquid occurring in a first wicking zone cannot be transported/wicked into a second wicking zone, thereby giving rise to a signal indicative of liquid in the second wicking zone. Thereby, it is assured that liquid occurring within a given wicking zone is also associated with said wicking zone upon detection in the monitor interface.

In an embodiment, transport of liquid between wicking zones of the plurality of wicking zones is inhibited by means of an embossed structure in the nonwoven substrate. For example, where the nonwoven substrate comprises meltable synthetic fibres, the embossed structure may be formed by heating the nonwoven substrate according to a layout of wicking zones, such that the localised heating of the fibres of the nonwoven substrate gives rise to the formation of borders, wherein liquid cannot be transported across by means of wicking/capillary action.

In embodiments, the one or more of the wicking zones are made of nonwoven material. Zones of the ostomy appliance that are not connected to the electrodes may be comprise a polymeric material, such as a polymeric film, and/or adhesive.

In embodiments, the nonwoven material is adapted to guide liquid towards the liquid detection zone by having a majority of the fibres in the nonwoven material aligned close to or along a line pointing towards the liquid detection zone.

In embodiments, one or more wicking zones are connected to electrodes in the liquid detection zone.

In an embodiment, the ostomy appliance comprises a wicking region and a sensing region, wherein the monitor interface is arranged in the sensing region. The sensing region may also be denoted the liquid detection zone. Thereby, the wicking region may be absent of electrodes, as these are associated with the sensing region only. This gives rise to a particularly robust ostomy appliance, as the risk of failure or rupture of the electrodes is minimised. The sensing region may be arranged along/on or in the vicinity of an outer periphery of the ostomy appliance. The sensing region may be arranged in a neck portion of the ostomy appliance. For example, the ostomy appliance may be substantially circular or elliptical, and the neck portion may extend radially away from the stomal opening (e.g., a centre point thereof), such that the neck portion is a portion extending further away from the ostomy appliance than the outer periphery of the generally circular/elliptical periphery.

In an embodiment, the nonwoven substrate covers the entirety of the distal side of the first adhesive layer. Thus, the first adhesive layer and the nonwoven substrate may have a substantially identical area. In other words, an inner (e.g., to accommodate a stomal opening) and outer periphery of the first adhesive layer may be aligned with a similar inner and outer periphery of the nonwoven substrate. In embodiments, the nonwoven substrate may be considered a substrate for the first adhesive layer.

In an embodiment, the first adhesive layer is provided on a proximal surface of the nonwoven substrate. Thereby, once liquid/moisture has absorbed through the first adhesive layer, it immediately reaches the nonwoven substrate wherein it may be transported/guided in the appliance plane to the monitor interface for detection.

In an embodiment, a backing layer is provided on the distal side of the nonwoven substrate. For example, the backing layer may be impermeable, such as fluid impermeable. For example, the backing layer may be a polymeric film, such as a thermoplastic polyurethane (TPU) film. Thereby, the backing layer may prevent evaporation during transport of liquid within the nonwoven substrate, thereby providing for a stronger signal at the monitor interface as a result of potentially more liquid reaching the monitor interface.

In an embodiment, the ostomy appliance is a base plate comprising a stomal opening and an outer boundary defining the outer edge of the base plate. The base plate may further comprise coupling means for coupling an ostomy bag to the base plate (two-piece), or the base plate may comprise an ostomy bag (one-piece).

In an embodiment, the ostomy appliance is a sensor patch for attachment to the adhesive surface of a base plate. The sensor patch may have a stomal opening and an outer boundary defining the outer edge of the sensor patch. Thereby, the ostomy appliance may be an accessory for a base plate, which is configured to be adhered/attached to the proximal adhesive surface of a generic/conventional base plate, such that the sensor patch is arranged in the interface between the skin surface of a user and the base plate.

In a second aspect of the invention, an ostomy system is disclosed. The ostomy system comprises an ostomy appliance according to embodiments of the first aspect of the invention and a monitor device couplable to the ostomy appliance. The monitor device comprises a processor, a memory and a first interface coupled to the processor and the memory. The first interface is further configured to couple to the monitor interface of the ostomy appliance. The monitor device is configured to measure an electrical quantity of the nonwoven substrate, such as by means of the sensing electrodes of the ostomy appliance.

The monitor device may further comprise a power unit, further sensors, a charging interface, and/or an interface for communication with an accessory device, such as a smart device (e.g., a smartphone, a tablet, or a smartwatch).

For example, the electrical quantity may be one or more of resistance, conductance, impedance, and capacitance. In other words, the monitor device may be configured to measure the resistance, the conductance, the impedance and/or the capacitance of the nonwoven substrate by means of the sensing electrodes of the ostomy appliance, which are connected to the monitor device in the coupled configuration via the terminals of the monitor interface. For example, the electrical quantity may be measured between two sensing electrodes. In other words, the monitor device may be configured to apply a voltage across two sensing electrodes.

In an embodiment, the monitor device is configured to, in accordance with the value of the electrical quantity being indicative of liquid in contact with one or more sensing electrodes of the plurality of sensing electrodes of the monitor interface, determine that liquid is present in the ostomy appliance.

For example, where the value of the resistance (or any of the other electrical quantities) is in noncompliance with a threshold (e.g., falls below a first threshold), it may be indicative of liquid in contact with one or more of the plurality of sensing electrodes of the monitor interface, and thus, the monitor device may determine that liquid is present in the ostomy appliance - in particular to a level where the liquid has absorbed through the first adhesive layer and reached the nonwoven substrate from where it has wicked to the monitor interface. Thus, the monitor device may at least determine that liquid is present in the nonwoven substrate, where the source of the liquid is likely the presence of liquid in the interface between the skin surface and the ostomy appliance.

In an embodiment, the monitor device is configured to, in accordance with determining that liquid is present in the ostomy appliance, transmit a first monitor device signal indicative of liquid being present in the ostomy appliance.

The first monitor device signal may be transmitted to an accessory device. For example, the monitor device may comprise an interface comprising a transceiver module connected to the processor and configured for connecting the monitor device to an accessory device of the ostomy system. In embodiments, the monitor device is configured to transmit monitor device signal(s). The interface may be configured as an accessory interface for connecting, e.g., wirelessly connecting, the monitor device to one or more accessory devices. The interface may comprise an antenna and a wireless transceiver, e.g., configured for wireless communication at frequencies in the range from 2.4 to 2.5 GHz. The wireless transceiver may be a Bluetooth transceiver, i.e., the wireless transceiver may be configured for wireless communication according to a Bluetooth protocol, e.g., Bluetooth Low Energy, Bluetooth 4.0, Bluetooth 5. The interface optionally comprises a loudspeaker and/or a haptic feedback element for provision of an audio signal and/or haptic feedback to the user, respectively.

Thereby, the accessory device may receive the first monitor device signal, process the signal, and issue a notification (e.g., in a visual interface of the accessory device, such as in a display) indicative of presence of liquid in ostomy appliance, whereby the user may take appropriate action.

In embodiments, the monitor device is configured to distinguish signals from a plurality of wicking zones as discussed above in relation to the first aspect of the invention, such that the monitor device may determine in what wicking zone of the plurality of wicking zones liquid is present, thereby providing more detailed information to the user about the presence of liquid.

It is envisioned that the invention as disclosed herein may also apply to wound dressings. For example, a wound dressing may be provided with a nonwoven substrate as disclosed, e.g., arranged on the distal side (e.g., the distal surface) of an absorbent core layer of the wound dressing, whereby liquid (e.g., sweat and/or exudate from the wound) may be transported in the nonwoven substrate to reach the monitor interface having sensing electrodes and corresponding terminals as disclosed.

### Detailed description of the drawings

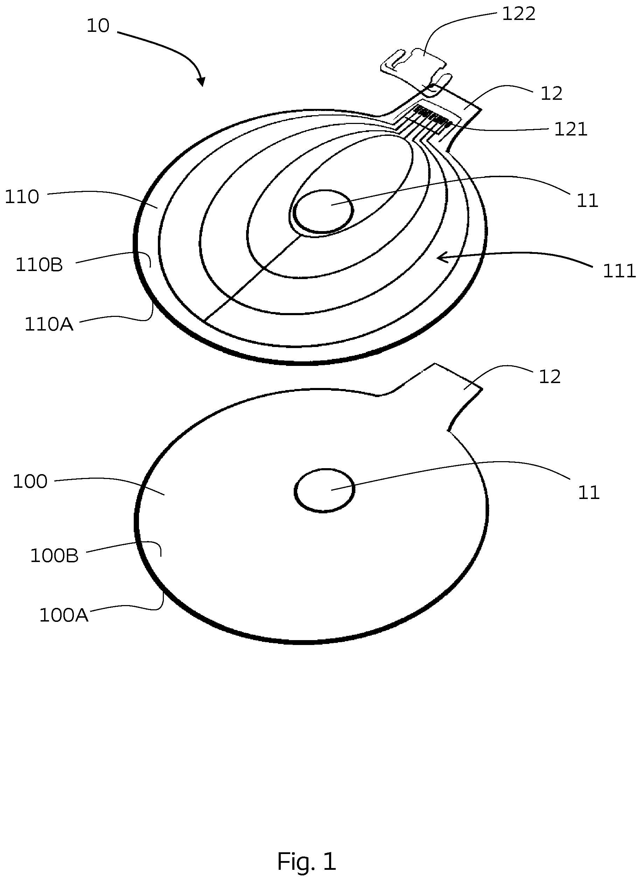

Fig. 1 illustrates an exemplary exploded perspective view of an ostomy appliance 10 according to an embodiment of the invention. The ostomy appliance 10 comprises a first adhesive layer 100 and a nonwoven substrate 110, both comprising a (coinciding) stomal opening 11 for receiving a stoma and a (coinciding) neck portion 12 extending radially away from the stomal opening 11.

The first adhesive layer 100 comprises a proximal surface 100A and a distal surface 100B. The proximal surface 100A is configured for attachment to the skin surface of a user. The nonwoven substrate 110 comprises a proximal surface 110A and a distal surface 110B. The nonwoven substrate 110 is arranged on the distal surface 100A of the first adhesive layer 100. A backing layer (not shown) may be arranged on the distal surface 110B of the nonwoven substrate 110, the backing layer being impermeable, such as liquid and/or moisture permeable, so as to inhibit evaporation of liquid in the nonwoven substrate 110.

The first adhesive layer 100 is moisture absorbing and configured to transport moisture absorbed on the proximal surface 100A to the distal surface 100B, where such moisture may be adsorbed by the nonwoven substrate 110 and further transported/wicked (i.e., in the appliance plane of the ostomy appliance) to a monitor interface 120. The monitor interface 120 comprises a plurality of terminals 121 and a connector 122 for providing a coupling to a monitor device (not shown). Each of the terminals of the plurality of terminals 121 are connected to a sensing electrode (not shown) in contact with the nonwoven substrate 110. The monitor interface 120 is arranged on the neck portion 12 of the ostomy appliance.

According to the illustrated embodiment, the nonwoven substrate 110 comprises a plurality of wicking zones 111, each being connected to one or more associated sensing electrodes of the monitor interface 120, such that liquid adsorbed in a given wicking zone may be transported, by wicking, to the one or more associated sensing electrodes of the given wicking zone. One or more, such as two, sensing electrodes, may be associated with one wicking zone.

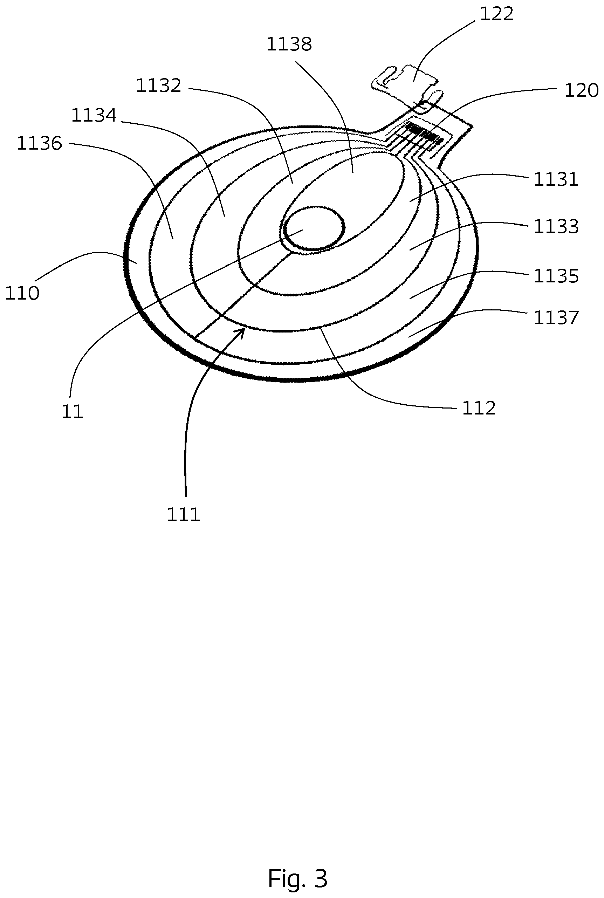

As shown in Fig. 1, the wicking zones 111 may have the shape of an ellipse cut along its major axis, thereby allowing a portion of each ellipse to communicate with a corresponding sensing electrode of the monitor interface 120. Details of the wicking zones 111 are provided in relation to Fig. 3.

The build of the ostomy appliance 10 provides for a robust device where liquid is transported, by wicking internally in the nonwoven substrate 110, to the sensing electrodes of the monitor interface 120. Thereby, risk of rupturing of electrodes or other failures relating to the sensing by means of electrodes is minimised.

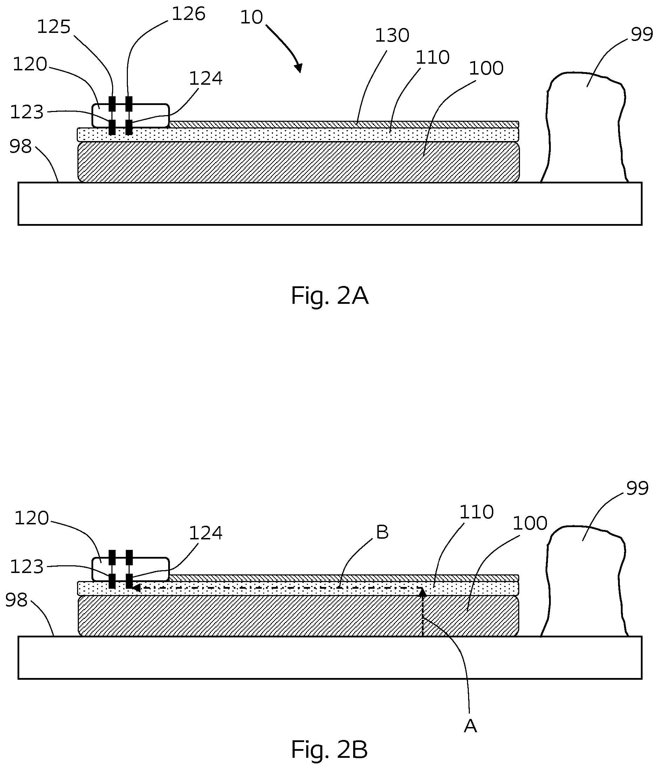

Fig. 2A illustrates an exemplary cross-sectional view of an ostomy appliance 10 according to an embodiment of the invention, the ostomy appliance 10 here being adhered to the skin surface 98 in the peristomal area of a stoma 99 (only left-hand side of the ostomy appliance 10 relative to the stoma 99 is shown).

The ostomy appliance 10 comprises the first adhesive layer 100, the nonwoven substrate 110 and a monitor interface 120. A backing layer 130 is provided on the distal surface of the nonwoven substrate. The backing layer 130 may prevent evaporation from the nonwoven substrate 110, and thus, the backing layer may be liquid and/or moisture impermeable.

The monitor interface 120 comprises at least two sensing electrodes, including a first electrode 123 and a second electrode 124. Further, the monitor interface 120 comprises at least two terminals including a first terminal 125 coupled to the first electrode 123 and a second terminal 126 coupled to the second electrode 124. The monitor interface 120 may further comprise coupling means, such as a connector 122 as shown in Fig. 1, for detachably fixating a monitor device to the monitor interface 120 and thus the ostomy appliance 10. The sensing electrodes 123,124 are in contact with the nonwoven substrate 110 such that a monitor device may measure an electrical quantity of the nonwoven substrate 110, e.g., resistance, conductance, impedance, and/or capacitance, via the terminals 125,126. In embodiments, the monitor interface is substantially planar. In embodiments, the electrodes 123,124 and the terminals 125,126 are combined in a single feature. For example, the monitor interface 120 may comprise planar pads for forming connections with a monitor device, where a proximal surface of the pad may be in contact with the nonwoven substrate 110 (thus constituting the sensing electrode) and a distal surface of the pad may constitute the terminal.

In embodiments, the nonwoven substrate 102 is shielded from the stoma 99 as such (e.g., by means of a liquid impermeable coating or similar), such that liquid from the stoma 99 may not directly enter the nonwoven substrate 102, meaning that the main access for liquid to enter the nonwoven substrate 102 is via absorption through the first adhesive layer 100 from the skin surface 98 (see Fig. 2B).

Fig. 2B illustrates the process of wicking and sensing at the monitor interface 120. Initially, a liquid appears in the interface between the skin surface 98 and the proximal surface 100A of the first adhesive layer 100. The liquid may be due to sweating, or it may be due to output from the stoma 98 propagating in the interface. The liquid will be absorbed by the first adhesive layer 100 and eventually reach the nonwoven substrate 110 and causing wetting thereof (dashed arrow A). Due to material properties of the nonwoven substrate 110, capillary action will cause the liquid to propagate/wick in the plane of the nonwoven substrate 110 (appliance plane) until it reaches the monitor interface 120 (dash-dotted arrow B). At the monitor interface, via the terminals and the sensing electrodes 123,124 in contact with the nonwoven substrate 110, a coupled monitor device (not shown) may detect a change in an electrical quantity. For example, where resistance is being monitored, the presence of liquid in the nonwoven substrate 110 will cause the resistance between the sensing electrodes 123,124 to decrease, thus constituting a signal indicative of liquid being present, and that the user might want to take appropriate action, e.g., to avoid an incident of leakage from the ostomy appliance. For example, the monitor device may issue/generate a monitor device signal indicative of liquid in the interface. The monitor device signal may be a wireless signal communicated to an accessory device, e.g., a smart device, where the user may receive a notification indicative of the presence of liquid.

Fig. 3 illustrates a perspective view of distal surface of the nonwoven substrate 110 with a plurality of wicking zones 111 bound by borders 112 (black lines) according to an embodiment of the invention. The plurality of wicking zones includes a first wicking zone 1131, a second wicking zone 1132, a third wicking zone 1133, a fourth wicking zone 1134, a fifth wicking zone 1135, a sixth wicking zone 1136, a seventh wicking zone 1137 and an eighth wicking zone 1138. Also illustrated is the monitor interface 120 and the connector 122.

The borders 112 may be formed in an embossing process, wherein the fibres of the nonwoven substrate 110 are heated/melted according to the desired pattern, thereby inhibiting capillary action across the border.

As illustrated, the wicking zones 111 may be formed as concentric ellipses with the stomal opening 11 in a focus or centre point thereof. The ellipses may be cut along a major axis (see, e.g., the second wicking zone 114), so as to double the number of wicking zones, in turn providing more precise indications of the presence of liquid.

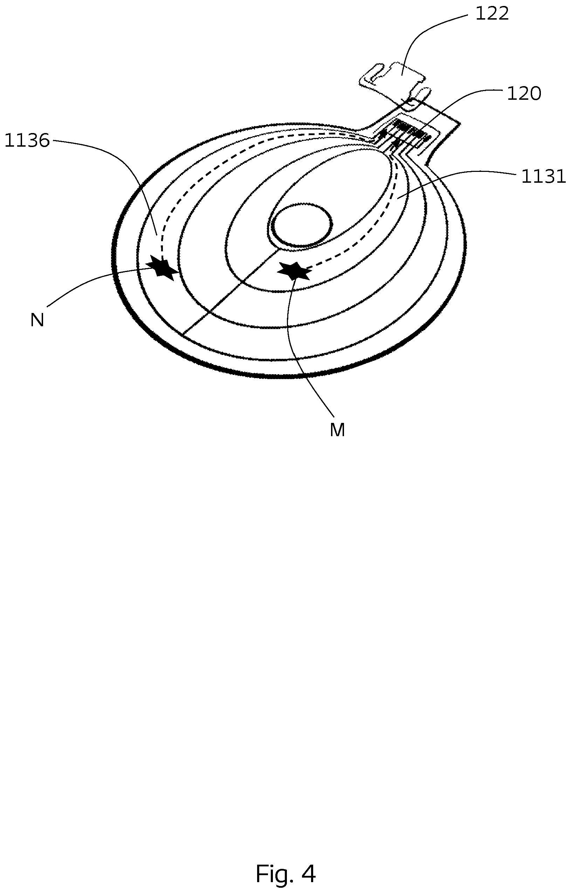

Each wicking zone of the plurality of wicking zones 111 is in communication with at least one corresponding/associated sensing electrode of the monitor interface 120. One or more sensing electrodes of the monitor interface 120 may be arranged in/associated with a given wicking zone. By being in communication with a corresponding sensing electrode is meant that liquid may wick from any point within the given wicking zone and reach the corresponding sensing electrode in contact with the same wicking zone (or more sensing electrodes of the given wicking zone). See also Fig. 4.

Fig. 4 illustrates an exemplary situation of two instances of liquid wetting the nonwoven substrate 110 in a first region M and a second region N. The first region M is arranged within the first wicking zone 1131 and the second region N is arranged within the sixth wicking zone 1136.

By means of capillary action in the nonwoven substrate 110, the liquid occurring in each of the two regions M,N is transported/wicked within the respective wicking zone 1131,1136 to the respective sensing electrode(s) of the monitor interface 120 (see dashed arrows in the respective wicking zones showing the path to the respective sensing electrodes). Thereby, a monitor device (not shown) coupled to the monitor interface 120 (e.g., detachably fixated via the connector 122) may determine that wetting has occurred within the first wicking zone 1131 and the sixth wicking zone 1136. Such information on the position of liquid may be valuable to the user to avoid future incidents of leakage.

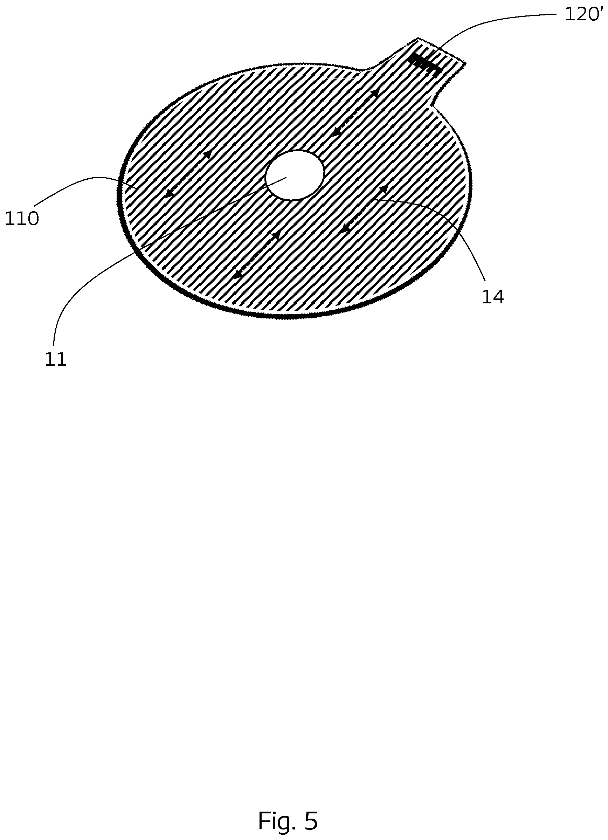

Fig. 5 illustrates the nonwoven layer 110 and the electrodes 120' in the monitor interface/liquid detection zone. The nonwoven layer 110 is adapted to guide liquid in two directions, namely towards and away from the liquid detection zone in the vicinity of, or defined by, the electrodes 120'. The nonwoven layer 110 is made of fibres and the fibres may be oriented such that they mainly extend towards the liquid detection zone. This means that not all the fibres are aligned completely but most fibres will be aligned close to a line pointing towards the liquid detection zone. All fibres may be aligned to in one direction but that is not required for having a preferred direction for liquid to propagate in. Liquid in the nonwoven material may be transported along the fibres of the nonwoven material. Thus, when most of the fibres are aligned the liquid will be propagated in the direction along the main fibre direction faster than in other directions. The main liquid propagation 14 when liquid is absorbed in the nonwoven layer 110 is illustrated by the arrows 14. Although particular features have been shown and described, it will be understood that they are not intended to limit the claimed invention, and it will be made obvious to those skilled in the art that various changes and modifications may be made without departing from the scope of the claimed invention. For example, it is envisioned that the sensor assembly and the electrodes thereof may be incorporated and/or provided in a variety of different embodiments without departing from the scope of the invention.

The specification and drawings are, accordingly, to be regarded in an illustrative rather than restrictive sense.

## Claims

1. An ostomy appliance (10) for attachment to the skin surface of a user, the ostomy appliance comprising:
- an adhesive layer (100) having a proximal side including a proximal surface (100A) for adhering the ostomy appliance (10) to the skin, and a distal side including a distal surface (100B);
- a plurality of electrical conductors (120) positioned in a liquid detection zone; and
- **characterised in that** a layer of nonwoven substrate (110) is arranged on the distal side of the adhesive layer, the layer of nonwoven substrate being liquid permeable and adapted to guide liquid towards said liquid detection zone.

2. The ostomy appliance according to claim 1, wherein the plurality of electrical conductors comprises a first electrode (123) and a second electrode (124) configured to detect presence of liquid in the nonwoven substrate (110).

3. The ostomy appliance according to claim 2 further comprising a plurality of terminals positioned in the liquid detection zone, the plurality of terminals comprising a first terminal (125) coupled to the first electrode (123) and configured to electrically couple with a corresponding terminal of a monitor device, and a second terminal (126) coupled to the second electrode (124) and configured to electrically couple with a corresponding terminal of the monitor device.

4. The ostomy appliance according to any of claims 2-3, wherein the nonwoven substrate is adapted to guide liquid towards said liquid detection zone by having a plurality of wicking zones (111) including a first wicking zone (1131), the first wicking zone being in communication with at least the first electrode (123), such that liquid within the first wicking zone may be guided to said first electrode.

5. The ostomy appliance according to claim 4, wherein transport of liquid between wicking zones (1131, 1132, 1133, 1134, 1135, 1136, 1137, 1138) of the plurality of wicking zones (111) is inhibited.

6. The ostomy appliance according to claim 5, wherein transport of liquid between wicking zones of the plurality of wicking zones is inhibited by means of an embossed structure in the nonwoven substrate.

7. The ostomy appliance according to any of claims 1-6, wherein the ostomy appliance comprises a wicking region and the liquid detection zone, wherein a monitor interface (120) is arranged in the liquid detection zone.

8. The ostomy appliance according to any of claims 1-7, wherein the nonwoven substrate covers the entirety of the distal side of the adhesive layer.

9. The ostomy appliance according to any of claims 1-8, wherein the adhesive layer is provided on a proximal surface (110A) of the nonwoven substrate.

10. The ostomy appliance according to any of claims 1-9, wherein a backing layer (130) is provided on the distal side of the nonwoven substrate.

11. The ostomy appliance according to any of claims 1-10, wherein the ostomy appliance is a base plate comprising a stomal opening (11) and an outer boundary defining the outer edge of the base plate.

12. The ostomy appliance according to any of claims 1-10, wherein the ostomy appliance is a sensor patch for attachment to the adhesive surface of a base plate.

13. An ostomy system comprising an ostomy appliance (10) according to any of claims 1-12, and a monitor device couplable to the ostomy appliance, the monitor device comprising a processor, a memory, and a first interface coupled to the processor and the memory, wherein the first interface is configured to couple to a monitor interface (120) of the ostomy appliance, and wherein the monitor device is configured to measure an electrical quantity of the nonwoven substrate (110).

14. The ostomy system according to claim 13, wherein the monitor device is configured to, in accordance with the value of the electrical quantity being indicative of liquid in contact with one or more of the electrical conductors of the plurality of electrical conductors of the liquid detection zone, determine that liquid is present in the ostomy appliance.

15. The ostomy system according to claim 14, wherein the monitor device is configured to, in accordance with determining that liquid is present in the ostomy appliance, transmit a first monitor device signal indicative of liquid being present in the ostomy appliance.

## Patentansprüche

1. Stomavorrichtung (10) zur Befestigung an der Hautoberfläche eines Benutzers, wobei die Stomavorrichtung Folgendes umfasst:
- eine Klebeschicht (100) mit einer proximalen Seite, die eine proximale Oberfläche (100A) zum Kleben der Stomavorrichtung (10) an die Haut einschließt, und einer distalen Seite, die eine distale Oberfläche (100B) einschließt;
- eine Vielzahl von elektrischen Leitern (120), die in einer Flüssigkeitsdetektionszone positioniert sind; und
- **dadurch gekennzeichnet, dass** auf der distalen Seite der Klebeschicht eine Schicht aus Vliessubstrat (110) angeordnet ist, wobei die Schicht aus Vliessubstrat flüssigkeitsdurchlässig ist und eingerichtet ist, um Flüssigkeit in Richtung der Flüssigkeitsdetektionszone zu führen.

2. Stomavorrichtung nach Anspruch 1, wobei die Vielzahl von elektrischen Leitern eine erste Elektrode (123) und eine zweite Elektrode (124) umfasst, die dazu ausgelegt sind, Anwesenheit von Flüssigkeit in dem Vliessubstrat (110) zu detektieren.

3. Stomavorrichtung nach Anspruch 2, ferner umfassend eine Vielzahl von Anschlüssen, die in der Flüssigkeitsdetektionszone positioniert sind, wobei die Vielzahl von Anschlüssen einen ersten Anschluss (125), der an die erste Elektrode (123) gekoppelt ist und dazu ausgelegt ist, elektrisch mit einem entsprechenden Anschluss einer Überwachungsvorrichtung zu koppeln, und einen zweiten Anschluss (126) umfasst, der an die zweite Elektrode (124) gekoppelt ist und dazu ausgelegt ist, elektrisch mit einem entsprechenden Anschluss der Überwachungsvorrichtung zu koppeln.

4. Stomavorrichtung nach einem der Ansprüche 2-3, wobei das Vliessubstrat dazu eingerichtet ist, Flüssigkeit in Richtung der Flüssigkeitsdetektionszone zu führen, indem eine Vielzahl von Dochtzonen (111) einschließlich einer ersten Dochtzone (1131) vorhanden ist, wobei die erste Dochtzone in Kommunikation mit mindestens der ersten Elektrode (123) steht, so dass Flüssigkeit innerhalb der ersten Dochtzone zu der ersten Elektrode geführt werden kann.

5. Stomavorrichtung nach Anspruch 4, wobei Transport von Flüssigkeit zwischen Dochtzonen (1131, 1132, 1133, 1134, 1135, 1136, 1137, 1138) der Vielzahl von Dochtzonen (111) verhindert wird.

6. Stomavorrichtung nach Anspruch 5, wobei Transport von Flüssigkeit zwischen Dochtzonen der Vielzahl von Dochtzonen mittels einer Prägestruktur in dem Vliessubstrat verhindert wird.

7. Stomavorrichtung nach einem der Ansprüche 1-6, wobei die Stomavorrichtung eine Dochtregion und die Flüssigkeitsdetektionszone umfasst, wobei eine Überwachungsschnittstelle (120) in der Flüssigkeitsdetektionszone angeordnet ist.

8. Stomavorrichtung nach einem der Ansprüche 1-7, wobei das Vliessubstrat die Gesamtheit der distalen Seite der Klebeschicht bedeckt.

9. Stomavorrichtung nach einem der Ansprüche 1-8, wobei die Klebeschicht auf einer proximalen Oberfläche (110A) des Vliessubstrats bereitgestellt ist.

10. Stomavorrichtung nach einem der Ansprüche 1-9, wobei eine Trägerschicht (130) auf der distalen Seite des Vliessubstrats bereitgestellt ist.

11. Stomavorrichtung nach einem der Ansprüche 1-10, wobei die Stomavorrichtung eine Basisplatte ist, die eine Stomaöffnung (11) und eine äußere Begrenzung umfasst, die den äußeren Rand der Basisplatte definiert.

12. Stomavorrichtung nach einem der Ansprüche 1-10, wobei die Stomavorrichtung ein Sensorpflaster zur Befestigung an der Klebefläche einer Basisplatte ist.

13. Stomasystem, umfassend eine Stomavorrichtung (10) nach einem der Ansprüche 1-12 und eine Überwachungsvorrichtung, die mit der Stomavorrichtung koppelbar ist, wobei die Überwachungsvorrichtung einen Prozessor, einen Speicher und eine erste Schnittstelle umfasst, die an den Prozessor und den Speicher gekoppelt ist, wobei die erste Schnittstelle dazu ausgelegt ist, mit einer Überwachungsschnittstelle (120) der Stomavorrichtung zu koppeln, und wobei die Überwachungsvorrichtung dazu ausgelegt ist, eine elektrische Größe des Vliessubstrats (110) zu messen.

14. Stomasystem nach Anspruch 13, wobei die Überwachungsvorrichtung dazu ausgelegt ist, gemäß dem Wert der elektrischen Größe, der Flüssigkeit in Kontakt mit einem oder mehreren der elektrischen Leiter der Vielzahl von elektrischen Leitern der Flüssigkeitsdetektionszone angibt, zu bestimmen, dass Flüssigkeit in der Stomavorrichtung anwesend ist.

15. Stomasystem nach Anspruch 14, wobei die Überwachungsvorrichtung dazu ausgelegt ist, entsprechend der Bestimmung, dass Flüssigkeit in der Stomavorrichtung anwesend ist, ein erstes Überwachungsvorrichtungssignal zu übertragen, das angibt, dass Flüssigkeit in der Stomavorrichtung anwesend ist.

## Revendications

1. Appareil de stomie (10) destiné à être fixé sur la surface cutanée d'un utilisateur, l'appareil de stomie comprenant :
- une couche adhésive (100) ayant un côté proximal comportant une surface proximale (100A) destiné à faire adhérer l'appareil de stomie (10) à la peau, et un côté distal comportant une surface distale (100B) ;
- une pluralité de conducteurs électriques (120) disposés dans une zone de détection de liquide ; et
- **caractérisé en ce qu'**une couche de substrat non tissé (110) est disposée sur le côté distal de la couche adhésive, la couche de substrat non tissé étant perméable aux liquides et adaptée pour guider le liquide vers ladite zone de détection de liquide.

2. Appareil de stomie selon la revendication 1, dans lequel la pluralité de conducteurs électriques comprend une première électrode (123) et une seconde électrode (124) configurées pour détecter la présence de liquide dans le substrat non tissé (110).

3. Appareil de stomie selon la revendication 2 comprenant en outre une pluralité de bornes positionnées dans la zone de détection de liquide, la pluralité de bornes comprenant une première borne (125) couplée à la première électrode (123) et configurée pour se coupler électriquement à une borne correspondante d'un dispositif de surveillance, et une seconde borne (126) couplée à la seconde électrode (124) et configurée pour se coupler électriquement à une borne correspondante du dispositif de surveillance.

4. Appareil de stomie selon l'une quelconque des revendications 2 à 3, dans lequel le substrat non tissé est adapté pour guider le liquide vers ladite zone de détection de liquide en ayant une pluralité de zones à effet de mèche (111) comportant une première zone à effet de mèche (1131), la première zone à effet de mèche étant en communication avec au moins la première électrode (123), de telle sorte que le liquide à l'intérieur de la première zone à effet de mèche puisse être guidé vers ladite première électrode.

5. Appareil de stomie selon la revendication 4, dans lequel le transport de liquide entre des zones à effet de mèche (1131, 1132, 1133, 1134, 1135, 1136, 1137, 1138) de la pluralité de zones à effet de mèche (111) est inhibé.

6. Appareil de stomie selon la revendication 5, dans lequel le transport de liquide entre des zones à effet de mèche de la pluralité de zones à effet de mèche est inhibé au moyen d'une structure gaufrée dans le substrat non tissé.

7. Appareil de stomie selon l'une quelconque des revendications 1 à 6, l'appareil de stomie comprenant une région à effet de mèche et la zone de détection de liquide, dans lequel une interface de surveillance (120) est agencée dans la zone de détection de liquide.

8. Appareil de stomie selon l'une quelconque des revendications 1 à 7, dans lequel le substrat non tissé recouvre la totalité du côté distal de la couche adhésive.

9. Appareil de stomie selon l'une quelconque des revendications 1 à 8, dans lequel la couche adhésive est fournie sur une surface proximale (110A) du substrat non tissé.

10. Appareil de stomie selon l'une quelconque des revendications 1 à 9, dans lequel une couche de support (130) est fournie sur le côté distal du substrat non tissé.

11. Appareil de stomie selon l'une quelconque des revendications 1 à 10, l'appareil de stomie étant une plaque de base comprenant une ouverture stomale (11) et une limite externe définissant le bord externe de la plaque de base.

12. Appareil de stomie selon l'une quelconque des revendications 1 à 10, l'appareil de stomie étant une pastille de capteur destinée à être fixée à la surface adhésive d'une plaque de base.

13. Système de stomie comprenant un appareil de stomie (10) selon l'une quelconque des revendications 1 à 12, et un dispositif de surveillance pouvant être couplé à l'appareil de stomie, le dispositif de surveillance comprenant un processeur, une mémoire et une première interface couplée au processeur et à la mémoire, dans lequel la première interface est configurée pour se coupler à une interface de surveillance (120) de l'appareil de stomie, et dans lequel le dispositif de surveillance est configuré pour mesurer une quantité électrique du substrat non tissé (110).

14. Système de stomie selon la revendication 13, dans lequel le dispositif de surveillance est configuré pour, en fonction de la valeur de la quantité électrique qui est révélatrice d'un liquide en contact avec un ou plusieurs des conducteurs électriques de la pluralité de conducteurs électriques de la zone de détection de liquide, déterminer que du liquide est présent dans l'appareil de stomie.

15. Système de stomie selon la revendication 14, dans lequel le dispositif de surveillance est configuré pour, en fonction de la détermination que du liquide est présent dans l'appareil de stomie, transmettre un premier signal de dispositif de surveillance révélateur de la présence de liquide dans l'appareil de stomie.
